# EUROPEAN PATENT SPECIFICATION

(11) **EP 3 938 017 B1**
(45) Date of publication and mention of the grant of the patent: **07.08.2024**
(21) Application number: 20770695.3
(22) Date of filing: 12.03.2020
(51) Int. Cl.: A61M 16/10, A61B 5/0205, A61M 16/20

(54) **OXYGEN MONITORING AND CONTROL SYSTEM**
SAUERSTOFFÜBERWACHUNGS- UND -STEUERUNGSSYSTEM
SYSTÈME DE SURVEILLANCE ET DE COMMANDE D'OXYGÈNE

(30) Priority: 12.03.2019 US 201962816974 P
(43) Date of publication of application: 19.01.2022
(73) Proprietor: Live Fully, Inc., Monument, Colorado 80132 (US)
(72) Inventor: COMMERFORD, Bernard Lawrence, Colorado Springs, Colorado 80921 (US); GUSTAVSSON, Bo David, Monument, Colorado 80132 (US); JANZEN, Dianne M., Monument, Colorado 80132 (US); MEESTER, Troy A., Colorado Springs, Colorado 80921 (US); SWANSON, Paul S., Monument, Colorado 80132 (US); SWIGRIS, Jeffrey J., Denver, Colorado 80238 (US); VIERZBA, Michael T., Colorado Springs, Colorado 80923 (US); VIERZBA, Thomas M., Monument, Colorado 80132 (US); WOOD, Donna J., Monument, Colorado 80132 (US)
(74) Representative: Kilburn & Strode LLP
(86) International application number: PCT/US2020/022437
(87) International publication number: WO 2020/186085

(56) References cited:
- WO-A1-2017/165749
- WO-A1-99/04841
- JP-A- 2014 124 343
- US-A1- 2010 224 192
- US-A1- 2010 224 192
- US-A1- 2018 304 038
- US-B2- 10 136 859

## Description

This application claims the benefit under 35 U.S.C. § 119(e) of United States Provisional Application Serial No. 62/816,974, entitled "OXYGEN MONITORING AND CONTROL SYSTEM," filed on March 12, 2019.

### FIELD

The disclosure relates to devices, systems, and methods for measuring and controlling blood oxygen in a subject.

### BACKGROUND

Long term oxygen therapy (LTOT) is required by close to six million people worldwide for a variety of medical reasons. There are four types of oxygen systems presently in common use.

The most common system involves larger home-based concentrators that take room air and through a process of compression and filtering remove nitrogen and "concentrate" oxygen. The home concentrator has two significant limitations: 1) It is by design not portable; it is heavy and requires direct connection to household voltage; 2) All controls are located on the concentrator itself, and the setting is fixed and not responsive to the actual needs of the user as their activity level changes throughout the day.

The second category involves small pre-filled tanks provided by a Durable Medical Equipment (DME) firm. Using tanks requires an oxygen -conserving device (regulator) that reduces the pressure from approximately 2000 pounds per square inch (psi) to a more workable 35 psi and delivers the oxygen in pulses in order to conserve the oxygen supply.

Liquid oxygen is highly regulated and hard to obtain due to expense. Liquid oxygen systems having a small refillable tank that is replenished from a larger storage dewar provide a limited amount of portable oxygen, and often require a small cart or other wheeled device to accommodate their size and weight. By volume, liquid oxygen provides a longer supply of oxygen than compressed oxygen.

Smaller, portable oxygen concentration (POC) devices include an electronic device that can be recharged from standard household or 12 -volt electrical sources.

The significant limitation on the POCs is the available flow rates are limited. Some offer pulse only while others also offer a continuous flow capability at lower settings. The available settings do not equate to flow rates on LPM. Instead, settings are simply machines settings with no correlation to flow rates. The POC provides a small "bolus" of oxygen at the beginning of the inhalation cycle, significantly different than the pulse received from a standard oxygen regulator used with a portable oxygen tank. POCs is leave the patient "air hungry" during periods of increased activity. The reason patients feel "air hungry" is that even at their highest settings, POCs can only deliver oxygen at approximately 30% FIO₂ (the FIO₂ of free air in the atmosphere is approximately 21%) versus oxygen from tanks that is delivered at close to 92% FIO₂.

In addition to problems associated with weight and portability, supplemental oxygen is expensive. Medicare will spend $2.6B this year on supplemental oxygen according to a GAO report. Many approaches have been developed to conserve oxygen. A subject's oxygen demand is usually determined by a medical professional in consultation with the subject based on the results of treadmill analysis, a timed walk, by reference to standard tables, or through the process of what is called a Titration test (this test is formalized for Continuous Positive Airway Pressure (CPAP) and a version of this test is used for various lung diseases. In other diseases, Idiopathic Pulmonary Fibrosis (IPF) for instance, the Technician walks with the patient and provides increasing flow rates of supplemental oxygen until the patient can walk and maintain a saturation level of at least 89%)

Typically, the oxygen flow rate is set to a value that may be adjusted in future analyses if the subject reports symptoms associated with inadequate oxygen (hypoxia). This type of flow rate represents a tradeoff between the increased demand required during physical exertion and the reduced demand required during sedentary periods and is usually too little for the former and too much for the later. If they choose a flow rate that is too high they waste supplemental oxygen; if they choose a flow rate that is too low, they damage their vital organs (brain, heart, etc.).

Some approaches to conserving oxygen take advantage of the breathing cycle (inspiration followed by exhalation) of the subject by delivering oxygen only at the start of each inhalation and not during the exhalation portion of the breathing cycle. These approaches require use of a detector to determine when inspiration commences. Pressure detectors in a nostril tube and chest straps have been described as suitable for these applications. These approaches constitute open -loop systems for conserving oxygen by timing the delivery of oxygen to the subject so that oxygen is "pulsed" to the subject only during inhalation and is not provided during exhalation. Such systems can provide an advantage over systems that rely on a set (continuous) flow rate in terms of lowering wasteful oxygen use, but do not accommodate physiological changes in oxygen demand based on the subject's true oxygen need as there is no "feedback" loop in existing and developed devices.

Systems regulating oxygen flow to the patient are disclosed in US Patent 10,136,859 and PCT Application WO 99/04841. However, neither document provides features that would be capable of automatically allowing high oxygen flow in the case of systemic malfunction.

### SUMMARY

The invention is defined by the appended claims. Subject-matter referred as embodiments and/or disclosures which are not covered by the claims is not part of the invention.

The disclosure provides a closed-loop solution to the problems associated with adjusting oxygen delivery in response to the subject's physiological demand in a way that overcomes many of the shortcomings of the existing technology.

The disclosure is directed to devices, systems and methods for providing closed loop control of delivery of oxygen (or oxygen enriched air) to a human subject who suffers from diminished lung capacity, cardio-pulmonary disease, or injury from over 200 various lung diseases. As described herein, such devices, systems and methods may provide the subject with a system for delivering adequate oxygen to maintain blood oxygen saturation under a variety of physiological and environmental conditions. Other applications of such devices, systems, and methods include, without limitation, modifying (lowering) the partial pressure of the oxygen content to the subject who may not suffer from diminished lung capacity, for applications such as sports training (high altitude simulation) or determining blood oxygen usage rates under physical stress. The devices can be used, for example, for subjects with reduced ability to process oxygen, who work in low oxygen environments (firefighters), and for athletes who pursue physical activities at altitudes above their acclimatized oxygen band.

Additional embodiments and features are set forth in part in the description that follows, and in part will become apparent to those skilled in the art upon examination of the specification or may be learned by the practice of the disclosed subject matter. A further understanding of the nature and advantages of the disclosure may be realized by reference to the remaining portions of the specification and the drawings, which forms a part of this disclosure.

In various other aspects, a method utilizing a Wi-Fi connection to automatically control the flow rate on a home/stationary concentrator to match the patient's need is provided. The methods, optionally using proprietary software, can allow the patient to control all aspects of the OFCU with their Smartphone. Methods can allow the patient to display important health and system information on their Smartwatch supporting both iOS and AOS. In various aspects, methods can use GNSS hardware to gather pertinent Air Quality Index information and couple that information with the temperature, humidity and barometric temperature to form a more robust data picture of the patient's operating environment. In various aspects, the OSCU, using the data gathering, data saving, and transmission of encrypted data, can facilitate Remote Patient Monitoring by the patient's medical team.

### BRIEF DESCRIPTION OF THE FIGURES

The Figures are meant for illustration only.
**Figure 1A** depicts a PID (proportional, integral, derivative) control loop, according to some variations of the disclosure;
**Figure 1B** depicts a block diagram of a closed loop oxygen delivery system, according to some variations of the disclosure;
**Figure 2** depicts a block diagram depicting details of an internal configuration of an oxygen flow control unit (OFCU) and its relationship with external components of the closed loop oxygen delivery system, according to some variations of the disclosure;
Figure 3A depicts the data display and control interfaces of an example OFCU, according to some variations of the disclosure;
Figure 3B depicts another example OFCU in a configuration attached to the oxygen regulator and affixed to a portable oxygen tank, according to some variations of the disclosure;
**Figure 4A** depicts the internal layout of the key components of the OFCU V1, according to some variations of the disclosure;
**Figure 4B** depicts the internal layout of the key components of an OFCU, according to the invention; and
**Figure 4C** depicts an external configuration of a multi-component OFCU, home/stationary concentrator mounted module, according to some variations of the disclosure.

### DETAILED DESCRIPTION

In various aspects, an OFCU configured to provide a closed loop oxygen supply system can be described comprising the following components: an oxygen source, an OFCU including a proportional valve coupled to a microprocessor that controls the communication system, which receives data from blood oxygen sensors. In various aspects, oxygen can be delivered to the user.

This system regulates the flow of oxygen to the subject through the proportional valve that calibrates the flow of oxygen to the subject. The OFCU provides components for inputting subject information such as the subject's desired target blood oxygen level. The target blood oxygen level and the actual blood oxygen level of the subject, as detected by the pulse/oximetry sensors, are used by the microprocessor to calculate the appropriate proportionally controlled valve settings required to deliver to the subject the necessary amount of oxygen to maintain the target oxygen level. When the sensors detect that the subject's blood oxygen levels are lower than the target oxygen level, the microprocessor signals the controller to increase flow through the controlled valve. When the blood oxygen levels are higher than the target oxygen level the microprocessor instructs the controller to decrease flow through the controlled valve.

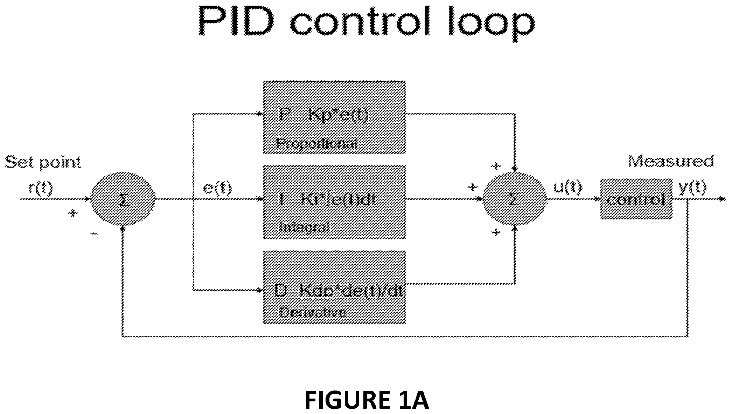

In variations as depicted in **Figure 1A****,** the processor is operating a PID (proportional, integral, derivative) control loop. The OFCU measures the oxygen levels and the processor calculates the difference between desired target oxygen levels and actual oxygen levels (Proportional). The processor also can calculate the velocity of the difference in levels (mathematical first Derivative). The processor also can calculate the long-term difference in target and actual values and integrates this small difference over time (Integral). If the subject changes from sedentary (sitting or lying down) to walking quickly, the blood oxygen deficit can change quickly. The first derivative portion of the control loop calculates the deficit will become very large very quickly and opens (or closes) the valve more aggressively Proportional portion of the algorithm would alone. If the subject is sedentary for a long time and the difference between target oxygen level and actual is slightly off, the mathematical integral of this difference will grow over time and cause further adjustments to the Oxygen flow rate to keep the error even lower. Because oxygen is already present in in the tube, the change in flow and flow rate can be instantaneous.

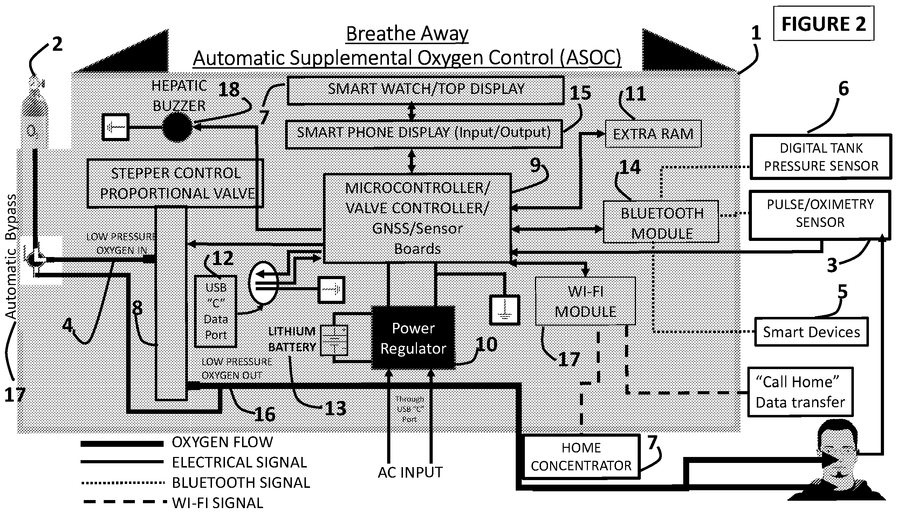

The OFCU may also calculate, record, and store data related to oxygen consumption, the subject's blood oxygen level overtime, respiration rate and other respiratory indicia. The subject or a healthcare professional can monitor oxygen use based on this data. In some aspects, as depicted in **Figure 2****,** the OFCU can be designed to be coupled to an oxygen tube between a low-pressure oxygen source and an oxygen delivery system. In this aspect the OFCU receives oxygen flowing from the oxygen source through the oxygen tube via an oxygen inlet port (4) attached to the proportional valve of the OFCU. The oxygen flow rate can be regulated by the proportional controlled valve and exits via an oxygen outlet port **(16).** The oxygen outlet port can be attached to an oxygen tube leading to an oxygen delivery system such as a transtracheal tube, a nasal cannula worn by the subject or a high-altitude training mask. The OFCU can be designed to be highly portable, transported in the subject's pocket or handbag or alternatively, connected to the oxygen regulator attached to the O2 tank, or small and light enough to attach to a subject's belt or clothing when in use.

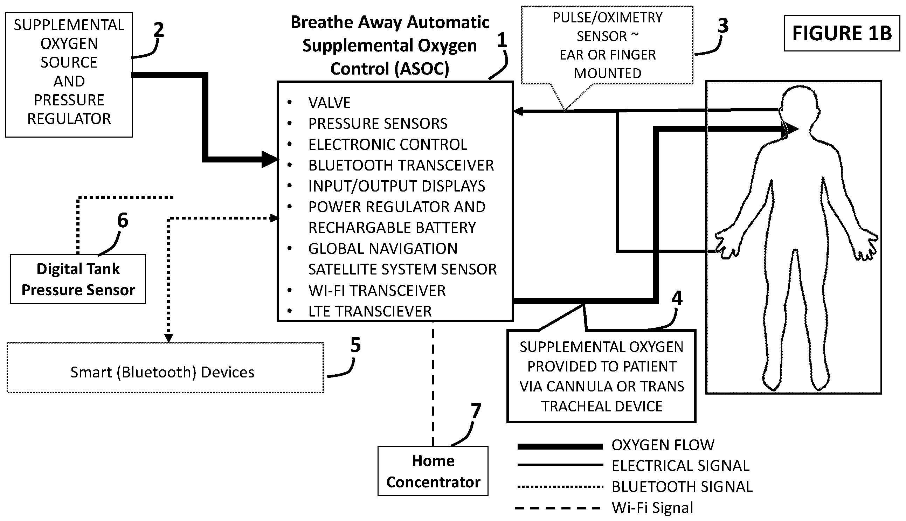

Various aspects of the disclosed OFCU are configured as a closed loop oxygen delivery system as depicted in **Figure 1B****.** In the figure, oxygen flows through the system from left to right along the path indicated by the heavy line, originating with an oxygen source comprising an oxygen reservoir or generator and a pressure regulator **(2).** The oxygen flows from the oxygen source to the OFCU **(1).** Oxygen then flows from the OFCU to the patient via an oxygen delivery device, for example a cannula or trans-tracheal device, as depicted in **(4).** A physiological monitoring device such as a pulse/oximetry sensor, shown in **(3),** monitors the level of oxygen present in the patient and relays this information to the OFCU via a wired or wireless connection to close the loop connecting oxygen delivery to oxygen demand as indicated by the heavy solid line. Additional wired or wireless inputs and outputs may transfer data to or from the OFCU from additional external devices (Smartphones and/or Smartwatches and Bluetooth-enabled hearing aids as shown in **(5)** by the dashed line. Additional information related to remaining oxygen in portable tanks is provided to the OFCU using a Bluetooth connection to the Digital Tank Pressure Sensor **(6).** This capability provides the patient with oxygen reserve time in minutes versus the current pounds per square inch (psi) that is of little use to patients. There is an additional wireless (Wi-Fi) connection between the OFCU and the patient's Home/stationary Concentrator **(7).** This provides control over the flow rate for supplemental oxygen from the Home/stationary Concentrator, a device that provides the majority of the patient's supplemental oxygen when they are home.

### Supplemental Oxygen Sources

In one aspect the oxygen source comprises a small compressed oxygen bottle sufficiently small and light to be readily portable. This bottle may be attached by a quick release pressure regulator such that the regulator can be easily and quickly removed from the tank and placed on a new tank once depleted. In some aspects the oxygen source may comprise a group of bottles with one or more pressure regulators arranged in such manner as to provide a low profile or to conform to the subject's back in a manner in which the subject can be spared injury should they fall or otherwise strike the oxygen source against a surface in the course of their activities. In other aspects the oxygen source may comprise a larger non-portable compressed oxygen source and regulator. In some aspects the oxygen source may comprise a liquid oxygen reservoir, which may be portable or non-portable and may further comprise a pressure regulator. In some aspects the oxygen source may be a large institutional liquid oxygen source such as may be found in a hospital, neighborhood based Urgent Care facility or an extended care facility such as a hospice center.

In other aspects the oxygen source may comprise a portable oxygen concentrator which may be battery powered. In other aspects the oxygen source may comprise a larger non-portable oxygen concentrator. Other oxygen production or enrichment systems familiar to those of skill in the art may be incorporated into the disclosure on the basis that all such systems deliver oxygen to the subject through a standard tubing system into which the OFCU and other components of the disclosed closed loop oxygen delivery system may be incorporated.

### Oxygen Flow Control Unit (OFCU)

The OFCU comprises the heart of the disclosed closed loop oxygen supply system. In some aspects the OFCU comprises a proportional controlled valve. In one aspect, the proportional ly-controlled valve provides an oxygen flow range of 0 -15 LPM across pressures ranging from atmospheric pressure to 50 psi. In some aspects the proportionally controlled valve provides an oxygen flow resolution of about 0.7 standard liters per minute (SLPM) per step with a step position resolution of about 0.001 inches. In some aspects the proportional controlled valve can be a low power valve. In some aspects the power consumption can be less than 4 watts nominal during adjustment with zero power consumption to maintain position. In some aspects the proportional control valve has a duty cycle (fully open to fully closed) response time of less than one second. In some aspects a valve controller can control the proportional control valve. In some aspects a microprocessor can actuate the controller.

Various alternative aspects of the OFCU (1) device are indicated within the shaded box within **Figure 2****.** External devices and connections are indicated outside of the shaded region. Within the figure, oxygen flow is depicted by the heavy black lines, electrical signals are depicted with a light black line, and wireless signals are depicted with dots for Bluetooth and dashes for Wi-Fi. The oxygen derived from an external source **(2)** flows to the patient depicted in the lower right corner of the figure. The oxygen first enters the OFCU **(1)** through a port **(4)** configured to fit a standard oxygen delivery tube and enters the proportional controlled valve **(8).** The proportional controlled valve regulates the flow rate of the oxygen transiting the valve body in response to inputs derived from a microcontroller control unit **(9).** The microcontroller control unit **(9)** receives power from a power regulator **(10).**

In some aspects the power regulator **(10)** may be powered by an integral battery **(13),** for example a lithium battery delivering 6,000 mAh at 5.0V. In some aspects of the disclosure the microcontroller control unit **(9)** can be powered by an external power source, such as an external charging device via USB-C (5V). The power regulator **(10)** can also be responsible for charging and regulating the discharge of the battery **(13).** The battery may also be charged via a USB-C port **(12),** which also allows two-way data transfer between external devices connected to the USB-C data port **(12)** and the microcontroller control unit **(9).** The USB-C data port **(12)** may also be used to update or modify the microprocessor control unit **(9)** firmware. The microcontroller control unit **(9)** can be in further communication with an integrated main display on the Version 1 device or a Smartphone **(15)** on Versions 2 device and the top display on Version 1 device or Smartwatches **(7)** on Version 2 devices, and the Bluetooth module **(14)** to allow Bluetooth communication with external devices **(5)** and may also provide a dedicated port for wired sensor inputs **(3)** such as pulse/oximetry sensors. The microcontroller control unit may also comprise additional random -access memory storage **(11)** for extended data logging and non-volatile storage of such data. The microcontroller control unit also may activate an audio, visual or haptic alarm **(18)** upon reaching certain system and user-defined alarm states.

In various other aspects the microprocessor control unit can communicate with sensors to adjust the valve settings to regulate the flow of oxygen to the subject based on the sensor input in a programmed manner to provide a closed loop system. In some aspects the amount of oxygen delivered by the system to the subject can be determined by the physiological demand of the subject as detected by the sensors. In some aspects the amount of oxygen delivered by the system to the subject can be determined by the amount of available oxygen in the oxygen source. The microprocessor control unit may be programmed to deliver oxygen at varying rates in response to any sensor input signal and can be programmed to evaluate different responses to various combinations of sensor input.

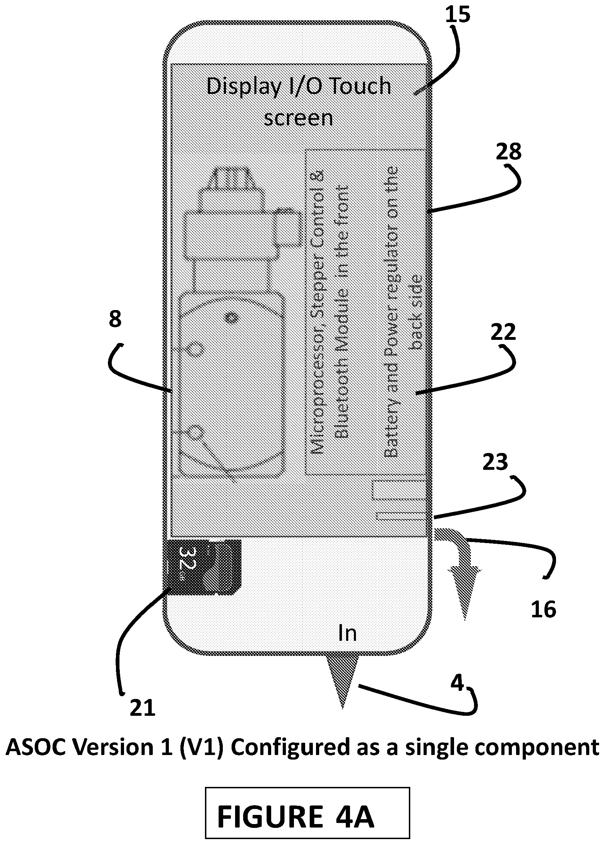

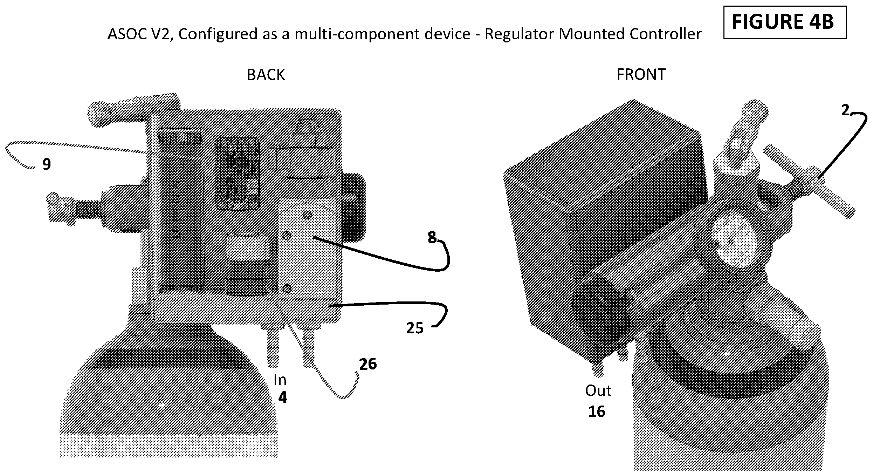

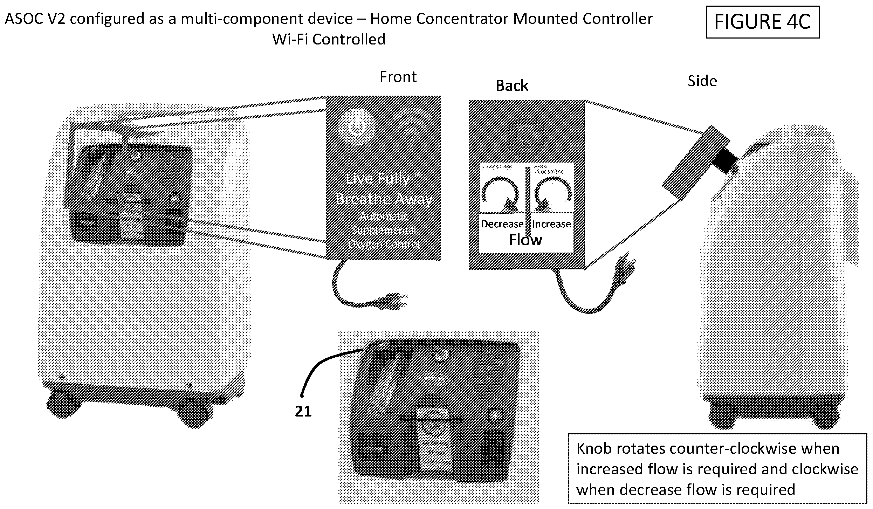

In some aspects the Signal Extraction Pulse Oximetry Platform unit comprises a 9 or 10 pin power and communications connector. In some aspects the Signal Extraction Pulse Oximetry Platform comprises a 20 or 25 pin sensor connector. In some aspects the Signal Extraction Pulse Oximetry Platform comprises a DC input voltage of about ±5% consuming 310 mW to 600 mW power. In some aspects the Signal Extraction Pulse Oximetry Platform comprises a serial interface with a variable baud rate of 9600 bps to 57,600 bps. In some aspects of the disclosure the Signal Extraction Pulse Oximetry Platform unit can be configured in a single stack or a double stack orientation. In some aspects of the disclosure the microprocessor control unit, Bluetooth module, Wi-Fi module and pulse/oximetry sensor control electronics are in a single stack configuration and in some aspects they are in a double stack configuration mounted on one side of a wafer and the battery on the opposite side, as depicted by **(22)** of **Figure 4****,** to provide thermal and electrical separation.

In various aspects the microprocessor control unit can execute programmed responses to sensor input data based on a plurality of executable modules. In some aspects the microprocessor can log sensor data. In some aspects the logged data may be retained in non-volatile memory. In some aspects the non-volatile memory may comprise a removable device. In some embodiments the logged data may be transferred wirelessly to an external computing device. In some aspects data can be transferred to a HIPAA certified external computing device via Bluetooth devices, Wi-Fi or LTE communication. In other aspects data may be transferred via a direct physical connection that may be established between the microprocessor and the external computing device via a cable or wire utilizing the USB-C connector. All data can be encrypted before being transmitted to any external destination.

The data captured can include pulse, saturation level, respiration rate, perfusion index, latitude, longitude, speed, altitude, humidity, barometric pressure, temperature and air quality index (AQI). The data is sent to the patient's medical team using the device's "Phone Home" feature to facilitate Remote Patient Monitoring (RPM). RPM is increasingly important attribute of modern medicine and a valuable tool during times of heightened concern about communicable diseases.

In one aspect the microprocessor communicates with output devices housed within the OFCU, which may comprise character or graphical LCD screens, light sources such as LEDs, as well as haptic or audible alarms. In some aspects the microprocessor communicates with external computing devices to receive instructions, display oxygen flow rates, pulse rates and perfusion index and to activate alarms or status indicators on such devices.

### Data Display and Control Elements of the OFCU

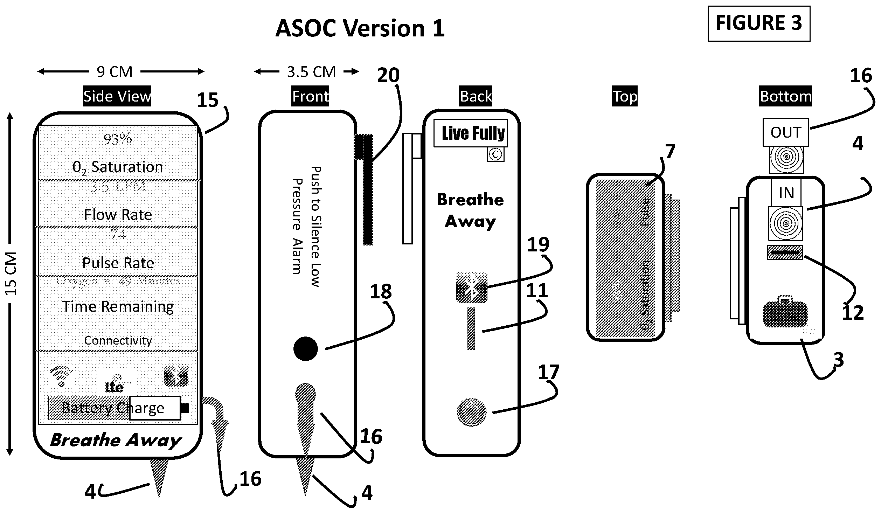

In one variation as depicted in Figure 3A, the OFCU can be approximately as tall 15 cm, and as wide 7.5 cm and approximately twice as thick 2 cm as a large cell phone.

In another variation depicted in Figure 4B, the OFCU can be approximately as tall as 12 cm, and as wide as 8 cm and as thick as 3 cm. In some variations the OFCU may be mounted semi-permanently to the oxygen regulator with a stainless-steel braided Teflon (or similar) hose **(24)** connecting the output port of the oxygen regulator and the input port of the OFCU manifold **(25).** In the present invention, the oxygen will enter the input port of the manifold which supports two valves. One valve controls the flow of oxygen in normal operation of the OFCU **(8).** The other valve is an automatic bypass valve **(26)** which is normally closed. However, in the event of a system malfunction, the automatic bypass valve **(26)** opens completely, allowing 100% of the flow rate in LPM the patient has set as the upper limit in flow rate on the oxygen regulator. In this variations, all control of the OFCU is managed through the use of an application on a Smartphone. In this variations, all important data is displayed on the patients Smartphone and their Smartwatch if they have authorized the connections.

In Reference to Figure 3A, the side view of the OFCU displays **(15)** the level of oxygen saturation reported by the sensor input and may also depict other programmed information such as remaining oxygen, pulse rate and battery status. The side panel may also provide a touch screen or other input device to allow the subject to input initial parameters to control the OFCU. On the back panel an on/off toggle button may be found, which may be lit to indicate status. The Emergency bypass function operates automatically in the unlikely event of any type of malfunction of the OFCU and provides the patient with 100% of the flow rate in LPM selected on the oxygen regulator.

In Figure 3A, the front of the OFCU may also possess an audible piezo buzzer **(18)** alarm and a reset button such as a soft button on the screen or Smartphone/Smartwatch to silence the alarm signals. The OFCU may possess a clip **(20)** for attachment to a belt or other article of clothing.

In Figure 3A, the bottom of the OFCU contains a connector for wired pulse/oximeter probe or similar sensor **(3).** The back of the OFCU provides a slot for accepting an SD card **(11).**

In Figure 3A, the top of the OFCU may repeat the side display **(7)** of oxygen saturation reported by sensor input and may also depict other programmed information such as remaining oxygen and battery status. The background color of this display will change as the quantity of remaining oxygen decreases.

In Figure 3A, at the bottom the OFCU houses the oxygen ports that provide oxygen into the OFCU **(4 for In and 16 for Out)** from the unit to the subject anda port for accepting sensor input (3) such as a20 or 25 pin jack commonly found on pulse/oximeter sensors as well as a USB-C port (12) for charging, data exchange, and firmware updates.

### Regulated Oxygen Delivery

In some aspects of the disclosure the regulated oxygen delivery system comprises a cannula or trans-tracheal tube. Other methods of oxygen delivery are known to those of skill in the art. The range of regulated oxygen delivery systems can be constrained by the ability of such systems to attach to the ports on the OFCU, which may be similar, if not identical, to the tubing attached to the oxygen source and are industry standard.

### Sensors

In some aspects **(****Figure 2****)** the sensors comprise a digital tank pressure sensor **(6)** for sensing the remaining available oxygen reserve. In some aspects the sensors comprise a global navigation satellite system (GNSS) that tracks navigation signals from the satellite systems operated by options such as the US Global Position System, the EU's Galileo navigation system, the Russian GLONASS navigation system and the Chinese BeiDou (Compass) navigation system , to provide location, altitude, and timing information. In some varations the sensor may measure the atmospheric pressure, temperature as well as humidity. In some aspects the sensors monitor the condition of the battery or other power source. In various aspects the sensors comprise physiological sensors capable of monitoring the subject's blood oxygen level (SpO2), pulse rate, and perfusion index. In some aspects the physiological sensor can be a pulse/oximeter. In some aspects the physiological sensor comprises a blood oxygen sensor - typically attached to the ear or finger of the subject that uses light (multiple wavelengths) to accurately sense blood oxygen levels of the subject.

In one aspect the pulse/oxygen sensor may be connected to the OFCU utilizing either a Bluetooth or wired connection. In some aspects the pulse/oximetry sensor may include an electronic control module that senses low perfusion and adjusts the sensor to provide a consistent signal to the microprocessor. In various embodiments, one or more sensors are configured to detect subject data accurately. When a subject is in motion, data from the subject can become inaccurate if the sensor is dislodged or moves. In various aspects, one or more sensors provided herein provide information that is accurate and related to motion. Examples of such sensors include the RD SET E1 sensor, and include Rainbow SET Board - MX-5 circuitboard by Masimo, or other sensors that are designed to be motion certified by the FDA to read in motion. In this aspect, the sensor input module may be configured to receive data from sensors designated to be motion certified, along with other functions including recording a subject's actual blood oxygen level and calculating a difference between the subject's actual blood oxygen level and the subject's target blood oxygen level.

The modular nature and variation of the disclosed device can allow as many forms of physiological sensors to provide input to the OFCU as possible. In some variations, the closed loop system can include sensors keyed to detecting leading indicators of oxygen demand (such as pulse rate) and can provide a distinct advantage over sensors measuring lagging indicators of oxygen demand (such as respiratory or percent blood oxygen level).

### Failsafe design of the OFCU

In various aspects of disclosure, the oxygen flow control unit can automatically bypass the microprocessor control of the proportional controlled valve. The OFCU verifies the health of the entire system every two seconds and if it determines there is any malfunction that would interrupt the flow of oxygen to the patient, the system automatically bypasses the closed loop oxygen delivery system and supplies oxygen to the subject directly from the oxygen regulator via the bypass valve **(26)** (Figure 4a). The bypass valve **(26)** is a normally open valve that is electrically kept in the closed position during routine operation, if an OFCU problem is detected, the power is removed from the valve and it moves to the fully open position.

As shown in **Figure 4A****,** the crucial mechanical part of the OFCU is the Control Proportionally Valve **(8)** which may comprise two pressure sensors embedded into its design providing tank side and patient side pressure measurements. The Automatic Bypass capability provides a smooth step back to the current state of the art in oxygen regulator control. The Automatic Bypass provides the liters per minute flow the subject has set as their personal worst-case scenario (pulse or continuous flow), on their oxygen regulator or concentrator.

In various aspects, as shown in **Figure 4B****,** the OFCU may be mounted and secured to the oxygen regulator. The merit of this approach relocates the majority of the weight of the OFCU to a pull cart location or to a backpack-carried oxygen tank. This aspect of disclosure also allows the patients Smartphone to control the OFCU receiving all important data/readouts and make any required changes to serve their needs using a proprietary software tool.

### EXAMPLES

The following Examples show aspects of the Oxygen Flow Control Unit. The Examples are illustrative of the disclosure and are not intended to limit the scope of the present invention which is defined in the appended claims.

### Example 1

The example Oxygen Flow Control Unit(OFCU) is a portable device configured to provide a closed loop oxygen delivery system by automatically adjusting the flow of oxygen to the subject through a proportional controlled valve in response to the oximetry readings derived from a pulse/oximetry sensor attached either at the Cavum Conchae (central part of the outer ear), forehead, foot, finger or other part of the subject. The Cavum Conchae is the preferred location as it is more sensitive to changes in oximetry, providing as much as 2 minutes notice versus finger located sensors, providing better predictive changes in flow rates. The second-best location is the ear lobe, also part of the body's core circulation. Additionally, the ear location can be hidden more easily by subjects and can be utilized, for example, in freezing weather (low perfusion) and wearing gloves and/or hats or caps.

Based on sensor input provided to the microprocessor and in conjunction with pre-programmed responses encoded in the microprocessor firmware, the microprocessor modifies the proportionally controlled valve settings to increase or decrease the flow of oxygen through the valve.

The OFCU is connected via standard oxygen tubing between the source of supplemental oxygen, either tank or concentrator (portable or home unit), and the person requiring supplemental oxygen. The OFCU utilizes the oxygen regulator present on oxygen tanks to reduce the pressure of the oxygen source from 400-2000 psi to a safer, low pressure (5-35 psi), delivered to the subject. When utilizing oxygen concentrators, the OFCU is connected via oxygen tubing between the concentrator and the subject. Lower pressure operation and commonly available tubing were selected because supplemental oxygen patients and their caregivers are familiar with these durable medical equipment (DME) supplies, and they are readily available. The OFCU may be connected to the subject utilizing either a cannula, a trans -tracheal device, or other devices known to those skilled in the art. The OFCU provides the subject with precise amounts of supplemental oxygen, in real time, whether the subject is utilizing pulse or continuous flow operation and may be used in lieu of pulse regulators (pulse regulators are 6 times more expensive than continuous flow regulators) as the OFCU automatically conserves oxygen while in operation.

The OFCU measures, controls, communicates and reports key health and system parameters to the subject (and their medical staff if desired). The oxygen sensor may also detect SpO2, pulse rate and perfusion index (PI). PI provides an evaluation of the quality of the signal and automatically adjusts the sensing parameters to provide quality data to the microprocessor. In various aspects of disclosure some OFCU models may also measure methemoglobin saturation, acoustical respiration rate, total hemoglobin, carboxyhemoglobin , Methemoglobin pulse co-oximetry, Pleth Variability Index, Respiration Rate, Desat 3D Alarm, Pi Delta Alarm, Plethysmograph Waveforms, Oxygen Reserve Index Signal Identification, Quality Indicator and oxygen content. These additional measurements are especially valuable for emergency transport situations or elite athletes and medical studies in a real-life setting (the subject "living their life, " not an artificial test as in a clinical setting), especially when the subject is active at higher altitudes or encountering contact with hazardous materials such as byproducts of uncontrolled fires.

When the subject first turns the unit on, the integrated input/output displays **Figure 2****, (15)** may query the subject if they are using an oxygen tank or a concentrator as their supplemental oxygen source. If the subject selects a tank, the tank digital pressure sensor **Figure 2****, (6)** is queried to determine the beginning pressure. This information will be used as part of the predictive supply management operation to keep the subject informed of remaining supplemental oxygen, a process that actively tracks initial tank pressure, current tank pressure and flow rate, and alerts the subject as the oxygen in the tank is depleted. The OFCU may alert smart devices the subject has paired with the OFCU (smart watches, fitness trackers and Bluetooth enabled hearing aids **Figure 2****, (5)),** as the quantity decreases from full to % to ½ to ¼ thereby helping the subject avoid running out of oxygen. There may also be an alarm **Figure 2****, (18)** that can alert the subject audibly if they have not paired any smart devices with the OFCU or a small vibrator to provide haptic notice. The display on the OFCU can also change its background color **Figure 2****, (7)** as the available oxygen decreases from full to % to ½ to ¼ remaining and may be equipped with a dedicated alarm light.

The subject can also be prompted to decide if they desire to have emergency personnel called if a subject's selectable preset parameter is breached, such as 55% blood oxygen saturation level and no movement sensed by the Global Navigation Satellite System (GNSS) sensor.

The subject will be asked what their desired minimum oxygen saturation level should be (88% is the lowest that may be selected as that is the generally agreed minimum safe level except in the Elite Athlete mode). The subject can also input their desired maximum heart rate during strenuous activity. The subject can be alerted to excursions below (or above for heart rate) preset limits audibly and visually on smart devices and reports generated from data logged during the period. The subject can be instructed as standard procedure to set their supplemental oxygen source regulator at the highest flow rate (in LPM) they have needed in the past. That is a key safety feature of the OFCU. In the event of battery failure or a malfunction of the OFCU, the Automatic Bypass feature bypasses all internal OFCU features and delivers 100% of the oxygen flow the subject set before that day's activities, giving them time to reach the source of their oxygen supply and manually set the flow rate actually required in their current situation (not the worst case scenario).

After those few user-selected settings are established, the user can begin their daily activities without requiring constant system monitoring. The OFCU will continually monitor their oxygen saturation, pulse and perfusion while automatically adjusting the flow of their supplemental oxygen, maintaining them at the desired level (+2%).

The OFCU stores pertinent data related to the activities of the patient if they desire. Such data may be stored locally and/or uploaded to other devices such as a cloud-based HIPAA storage system for review or sharing with medical personnel. Data stored and transmitted by the OFCU may include, without limitation, in graphical and tabular formats: pulse readings, oxygen saturation levels, oxygen flow rates, as well as speed traveled/elevation (above/below sea level) and the air quality index (AQI) for their location and time as determined by the GNSS module. The tabular format includes the number of events where the subject exceeded the parameters they selected during setup (pulse-high or low and highest and lowest measurement, oxygen saturation-high or low and highest and lowest measurement and averages for both). All of the data can be graphically displayed with a data point gathered every few seconds. All reports can be produced in a format that can be printed or emailed to the subject's medical team. The 32 GB SD Card can be sufficient to store eight years of reports for comparison purposes and for later investigation/sharing with the subject's medical team.

### Example 2

Subject blood oxygen saturation was maintained during the transition from sedentary to active states. A 70-year old male subject suffering from idiopathic pulmonary fibrosis (IPF) was fitted with an OFCU connected to a high-pressure portable oxygen bottle via 4 feet of tubing to the OFCU, which was in turn connected to a 4-foot-long nasal cannula oxygen delivery system to form a closed -loop oxygen delivery system. The OFCU included a MASIMO RD SET-E1 pulse/ox sensor attached to the subject at the subjects' Cavum Conchae. The OFCU was programmed to maintain the subject's oxygen saturation level at 91% regardless of the level of activity, and the oxygen regulator was set to 6 LPM pulse. This flow rate would be very wasteful without using the OFCU, because it is more than the subject needs while sedentary; however, it is the correct flow rate during times of increased/vigorous activity such as walking up an incline.

During a sedentary test period (sitting) the subject's observed blood oxygen saturation level was held at a constant 91% ± 1%, while during the active test period (vigorous walking on an incline between 6800 feet and 7500 feet above sea level) blood oxygen saturation varied between 89% - 92%. Under similar conditions, while using supplemental oxygen set to the prescribed flow rate but without use of the OFCU, the subject experienced a sedentary blood oxygen saturation of 82% - 89% and an active blood oxygen saturation variance of 72% - 92%.

### Example 3

Oxygen duration charts **(Table 2)** show that the standard M9/C oxygen tank (255 liters) lasts 2.5 hours when used at 4 LPM (pulse). Organizational testing has been completed on the Inogen One G5 using a single battery at an altitude of 7500 above sea level. Additional data has been gathered from the G5's Technical Manual (See ***Table 1*** below) related to the size of the bolus at different settings.

The following table summarizes the nominal bolus volumes (+/- 15%) delivered by the Inogen One G5 at 20C and sea level:

**Table 4: Source of the table: INOGEN ONE GS OXYGEN CONCENTRATOR TECHNICAL MANUAL 96-09302-00-01 Revision A, Page 5 of 18**

| **Flow Setting** | **Flow rate (ml/min)** | **10 BPM (ml/bolus)** | **17 BPM (ml/bolus)** | **25 BPM (ml/bolus)** | **30 BPM (ml/bolus)** |
|---|---|---|---|---|---|
| 1 | 210 | 21 | 12 | 8 | 7 |
| 2 | 420 | 42 | 25 | 17 | 14 |
| 3 4 | 630 840 | 63 84 | 37 48 | 25 34 | 21 28 |
| 5 | 1050 | 105 | 62 | 42 | 35 |
| 6 | 1260 | 126 | 74 | 50 | 42 |

Many of the portable oxygen concentrators do not in fact deliver oxygen in a relationship of 1 = 1 LPM, 2 = 2 LPM. The G5 effectively provides 1 LPM continuous at a setting of 6 with 17 breaths/minute.

Added for comparison and to provide a level playing field for all sources of portable oxygen, the liquid oxygen portable reservoir from Puritan-Bennet, Companion 500, was added to ***Table 2*** below. Testing has shown that using an M9/C tank set to 4 Lpm (pulse) when used in connection with the OFCU (normal daily activities) consistently lasts over 8 hours by conserving oxygen when the user is sedentary and providing sufficient oxygen when activities levels are higher, resulting in a user who is better served with proper oxygen to their brain and critical organs (heart, etc.). The device with the highest performance at each flow rate is shown with a black background and white numbers.

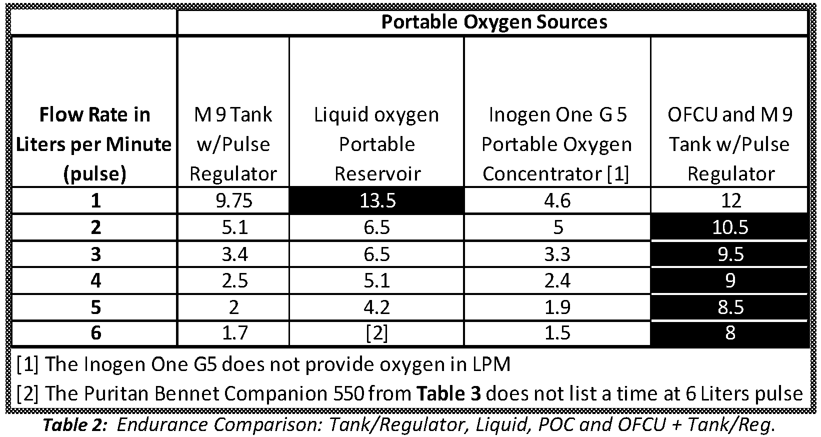

### Example 4

The OFCU may function in an emergency medicine support role, wherein multi-frequency blood sensors coupled to the OFCU capable of real -time detection of circulating specific hemoglobin levels in a subject can be used to monitor a patient for internal bleeding. In other cases, a detector capable of real -time monitoring of specific carbon monoxide levels can be coupled to the OFCU to provide supplemental oxygen to first responders or patients exposed to combustion products that impact their ability to absorb oxygen. In addition, real time detectors of methemoglobin (a side effect of many drugs used by hospitals and first responders) can be monitored through the OFCU and supplemental oxygen can be data to patients as necessary. Respiratory rate can be monitored by real -time analysis of pressure in the oxygen delivery tube connected to the OFCU to provide a log of a patient's respiration. The portable, low profile, data logging, closed -loop system represented by the OFCU increases the amount of actionable data delivered to the medical team serving a patient and can be transmitted while in route to the receiving medical facility, so they have high quality, specific data for this particular patient.

The OFCU may also find utility in military operations. In particular, the system provides vital remote data monitoring functions in Chemical, Biological, Nuclear and Radiological and Explosive (CBNRE) scenarios. Using telemetry to transmit the encrypted data to a command location while also ensuring reliable, appropriate oxygen saturation levels to the participant can be a capability multiplier. The future battlefield can include many combatants using exoskeletons to assist individuals carrying the extreme weight of communications and navigation equipment and munitions; however, such exoskeletons also inhibit the body's capability to dissipate heat, exacerbating the user's need for supplemental oxygen. The OFCU reads and reacts to this user's needs and delivers precise amounts of supplemental oxygen to keep the military member functioning at their peak while providing high quality, specific medical data to secure radio transmitting equipment, giving commanders actionable information.

It will be recognized that any variation disclosed herein can be combined with any other variation, whether or not such variations are described together or separately.

The invention is defined in independent claim 1. Preferred embodiments are matter of the appended dependent claims.

## Claims

1. An Oxygen Flow Control Unit (OFCU) configured to provide a closed loop oxygen supply system for maintaining a subject's blood oxygen level, the OFCU comprising:
a manifold (25) supporting two valves: a control valve (8) controlling oxygen flow during normal operation of the OFCU, and an automatic bypass valve (26) being normally closed, wherein, in the event of a systemic malfunction, the automatic bypass valve opens completely;
an oxygen tank fluidly connected to an oxygen regulator configured to reduce the oxygen pressure to 34-241 kPa (5-35 psi), the regulator fluidly connected to an oxygen inlet port (4) of the manifold;
a computing device comprising at least one processor electrically connected to a valve controller and to a power regulator;
one or more communication devices connecting the computing device to one or more sensors;
a plurality of modules executable by the at least one processor, the modules comprising:
a data input module configured to record a subject's target blood oxygen level;
a sensor input module configured to:
record a subject's actual blood oxygen level, and
calculate a difference between the subject's actual blood oxygen level and the subject's target blood oxygen level;
a control module configured to:
instruct the valve controller to increase oxygen flow from the oxygen inlet port of the control valve to an oxygen outlet port of the control valve when the subject target blood oxygen level is less than subject's lower threshold desired blood oxygen level;
instruct the valve controller to decrease oxygen flow from the oxygen inlet port to the control valve to an oxygen outlet port of the control valve when the subject's target blood oxygen level is greater than subject's upper threshold blood oxygen level; and
a regulated oxygen delivery system fluidly connected to the oxygen outlet port of the control valve.

2. The OFCU of claim 1, wherein the control valve is a proportional controlled valve.

3. The OFCU of claim 1, wherein the control valve is an electrically controlled valve.

4. The OFCU of claim 1, wherein the control valve controller reacts to inputs as directed by the microprocessor acting through the controller.

5. The OFCU of claim 1, wherein the power regulator is powered by an integral battery.

6. The OFCU of claim 1, wherein the communication device comprises a Bluetooth connection, a Wi-Fi connection, or an LTE connection.

7. The OFCU of claim 1, wherein the data input module is a touch screen module (15).

8. The OFCU of claim 1, wherein the sensor input module is configured for wired and/or Bluetooth connectivity.

9. The OFCU of claim 1, where the control valve is controlled by a closed loop algorithm to meter supplemental oxygen based on need as measured by a blood oxygen saturation sensor attached to the ear.

10. The OFCU of claim 1, wherein one or more sensors configured to accurately detect data from a subject while in motion.

11. The OFCU of claim 9, where the closed loop control algorithm utilizes heart rate, respiratory rate, or perfusion index as input parameter to control the control valve.

## Patentansprüche

1. Sauerstoff-Durchflussüberwachungseinheit (OFCU), die so ausgelegt ist, dass sie ein Closed-Loop-Sauerstoffversorgungssystem zum Aufrechterhalten des Blutsauerstoffgehalts eines Probanden bereitstellt, wobei die OFCU umfasst:
einen Verteiler (25), der zwei Ventile trägt: ein Steuerventil (8), das den Sauerstoffdurchfluss während des Normalbetriebs der OFCU steuert, und ein automatisches Bypassventil (26), das normalerweise geschlossen ist, wobei sich das automatische Bypassventil im Falle einer Systemstörung vollständig öffnet;
einen Sauerstofftank, der mit einem Sauerstoffregler in Fluidverbindung steht, der so ausgelegt ist, dass er den Sauerstoffdruck auf 34 bis 241 kPa (5 bis 35 psi) reduziert, wobei der Regler mit einem Sauerstoffeinlassanschluss (4) des Verteilers in Fluidverbindung steht;
eine Computervorrichtung, die mindestens einen Prozessor umfasst, der elektrisch mit einer Ventilsteuerung und einem Leistungsregler verbunden ist;
eine oder mehrere Kommunikationsvorrichtungen, die die Computervorrichtung mit einem oder mehreren Sensoren verbinden;
eine Mehrzahl von Modulen, die von dem mindestens einen Prozessor ausgeführt werden können, wobei die Module umfassen:
ein Dateneingabemodul, das zum Aufzeichnen eines Ziel-Blutsauerstoffgehalts eines Probanden ausgelegt ist;
ein Sensoreingabemodul, das ausgelegt ist zum: Aufzeichnen des tatsächlichen Blutsauerstoffgehalts eines Probanden;
Berechnen einer Differenz zwischen dem tatsächlichen Blutsauerstoffgehalt des Probanden und dem Ziel-Blutsauerstoffgehalt des Probanden;
ein Steuermodul, das ausgelegt ist zum:
Anweisen der Ventilsteuerung, den Sauerstoffdurchfluss vom Sauerstoffeinlassanschluss des Steuerventils zu einem Sauerstoffauslassanschluss des Steuerventils zu erhöhen, wenn der Ziel-Blutsauerstoffgehalt des Probanden unter dem unteren Schwellenwert des gewünschten Blutsauerstoffgehalts des Probanden liegt;
Anweisen der Ventilsteuerung, den Sauerstoffdurchfluss vom Sauerstoffeinlassanschluss des Steuerventils zu einem Sauerstoffauslassanschluss des Steuerventils zu reduzieren, wenn der Ziel-Blutsauerstoffgehalt des Probanden über dem oberen Schwellenwert des Blutsauerstoffgehalts des Probanden liegt;
wobei ein geregeltes Sauerstoffzufuhrsystem mit dem Sauerstoffauslassanschluss des Steuerventils in Fluidverbindung steht.

2. OFCU nach Anspruch 1, wobei das Steuerventil ein proportionalgesteuertes Ventil ist.

3. OFCU nach Anspruch 1, wobei das Steuerventil ein elektrisch gesteuertes Ventil ist.

4. OFCU nach Anspruch 1, wobei die Steuerventilsteuerung auf Eingaben reagiert, wie vom Mikroprozessor angewiesen, der über die Steuerung wirkt.

5. OFCU nach Anspruch 1, wobei der Leistungsregler von einer integrierten Batterie gespeist wird.

6. OFCU nach Anspruch 1, wobei die Kommunikationsvorrichtung eine Bluetooth-Verbindung, eine Wi-Fi-Verbindung oder eine LTE-Verbindung umfasst.

7. OFCU nach Anspruch 1, wobei das Dateneingabemodul ein Touchscreen-Modul (15) ist.

8. OFCU nach Anspruch 1, wobei das Sensoreingabemodul für drahtgebundene und/oder Bluetooth-Konnektivität ausgelegt ist.

9. OFCU nach Anspruch 1, wobei das Steuerventil durch einen Closed-Loop-Algorithmus gesteuert wird, um zusätzlichen Sauerstoff je nach Bedarf zu dosieren, der durch einen am Ohr angebrachten Blutsauerstoffsättigungssensor gemessen wird.

10. OFCU nach Anspruch 1, wobei ein oder mehrere Sensoren so ausgelegt sind, dass sie Daten von einem Probanden, während er in Bewegung ist, genau erfassen.

11. OFCU nach Anspruch 9, wobei der Closed-Loop-Algorithmus die Herzfrequenz, die Atemfrequenz oder den Perfusionsindex als Eingabeparameter zum Steuern des Steuerventils verwendet.

## Revendications

1. Unité de contrôle de débit d'oxygène (OFCU) configurée pour fournir un système d'alimentation en oxygène à boucle fermée destiné à maintenir le niveau d'oxygène sanguin d'un sujet, l'OFCU comprenant :
un collecteur (25) supportant deux vannes : une vanne de réglage (8) réglant le débit d'oxygène pendant le fonctionnement normal de l'OFCU, et une vanne de dérivation automatique (26) normalement fermée, la vanne de dérivation automatique s'ouvrant complètement en cas de dysfonctionnement systémique ;
un réservoir d'oxygène raccordé fluidiquement à un régulateur d'oxygène configuré pour réduire la pression d'oxygène à 34-241 kPa (5-35 psi), le régulateur étant raccordé fluidiquement à un orifice d'entrée d'oxygène (4) du collecteur ;
un dispositif informatique comprenant au moins un processeur relié électriquement à un contrôleur de vanne et à un régulateur de puissance ;
un ou plusieurs dispositifs de communication connectant le dispositif informatique à un ou plusieurs capteurs ; une pluralité de modules exécutables par l'au moins un processeur, les modules comprenant :
un module d'entrée de données configuré pour enregistrer un niveau d'oxygène sanguin cible du sujet ;
un module d'entrée de capteur configuré pour : enregistrer un niveau d'oxygène sanguin réel du sujet, et
calculer une différence entre le niveau d'oxygène sanguin réel du sujet et le niveau d'oxygène sanguin cible du sujet ;
un module de commande configuré pour :
donner l'instruction au contrôleur de vanne d'augmenter le débit d'oxygène depuis l'orifice d'entrée d'oxygène de la vanne de réglage jusqu'à un orifice de sortie d'oxygène de la vanne de réglage quand le niveau d'oxygène sanguin cible du sujet est inférieur à un niveau d'oxygène sanguin seuil inférieur souhaité du sujet ;
donner l'instruction au contrôleur de vanne de diminuer le débit d'oxygène depuis l'orifice d'entrée d'oxygène de la vanne de réglage jusqu'à un orifice de sortie d'oxygène de la vanne de réglage quand le niveau d'oxygène sanguin cible du sujet est supérieur à un niveau d'oxygène sanguin seuil supérieur du sujet ; et
un système de distribution d'oxygène régulé raccordé fluidiquement à l'orifice de sortie d'oxygène de la vanne de réglage.

2. OFCU selon la revendication 1, dans laquelle la vanne de réglage est une vanne à commande proportionnelle.

3. OFCU selon la revendication 1, dans laquelle la vanne de réglage est une électrovanne.

4. OFCU selon la revendication 1, dans laquelle le contrôleur de vanne de réglage réagit à des entrées selon les instructions du microprocesseur agissant par l'intermédiaire du contrôleur.

5. OFCU selon la revendication 1, dans laquelle le régulateur de puissance est alimenté par une batterie intégrée.

6. OFCU selon la revendication 1, dans laquelle le dispositif de communication comprend une connexion Bluetooth, une connexion Wi-Fi, or une connexion LTE.

7. OFCU selon la revendication 1, dans laquelle le module d'entrée de données est un module à écran tactile (15) .

8. OFCU selon la revendication 1, dans laquelle le module d'entrée de capteur est configuré pour une connectivité filaire et/ou Bluetooth.

9. OFCU selon la revendication 1, dans laquelle la vanne de réglage est commandée par un algorithme à boucle fermée pour mesurer de l'oxygène supplémentaire sur la base d'un besoin tel que mesuré par un capteur de saturation en oxygène sanguin attaché à l'oreille.

10. OFCU selon la revendication 1, dans laquelle un ou plusieurs capteurs sont configurés pour détecter avec précision des données auprès d'un sujet en mouvement.

11. OFCU selon la revendication 9, dans laquelle l'algorithme de commande à boucle fermée utilise la fréquence cardiaque, le rythme respiratoire ou l'indice de perfusion comme paramètre d'entrée pour commander la vanne de réglage.
